# EUROPEAN PATENT SPECIFICATION

(11) **EP 2 414 034 B1**
(45) Date of publication and mention of the grant of the patent: **18.06.2014**
(21) Application number: 09842772.7
(22) Date of filing: 30.10.2009
(51) Int. Cl.: A61N 1/05, A61N 1/375

(54) **AN IMPLANTABLE MRI COMPATIBLE MEDICAL LEAD WITH A ROTATABLE CONTROL MEMBER**
IMPLANTIERBARE MRT-KOMPATIBLE MEDIZINISCHE LEITUNG MIT DREHBAREM KONTROLLELEMENT
SONDE MÉDICALE IMPLANTABLE COMPATIBLE AVEC UNE IRM ET MUNIE D'UN ÉLÉMENT ROTATIF DE COMMANDE

(30) Priority: 31.03.2009 WO PCT/SE2009/000169; 10.07.2009 WO PCT/SE2009/000364
(43) Date of publication of application: 08.02.2012
(73) Proprietor: St. Jude Medical AB, 175 84 Järfälla (SE); Olsen, Linn, 186 95 Vallentuna (SE)
(72) Inventor: FORSLUND, Michael, S-112 46 Stockholm (SE); DJURLING, Henrik, S-175 65 Järfälla (SE); FORSSTRÖM, Patrik, S-175 65 Järfälla (SE); HENRIQUEZ, Leda, S-162 46 Vällingby (SE); DAHLBERG, Kenneth, S-112 26 Stockholm (SE); STEGFELDT, Olof, S-138 37 Älta (SE); SIVARD, Åke, S-171 70 Solna (SE)
(74) Representative: Sjölander, Henrik
(86) International application number: PCT/SE2009/000480
(87) International publication number: WO 2010/114433

(56) References cited:
- EP-A2- 0 709 111
- WO-A1-2006/093685
- US-A- 4 463 765
- US-A- 4 667 686
- US-A- 5 741 321
- US-A1- 2002 183 820
- US-A1- 2004 068 299
- US-A1- 2005 090 884
- US-A1- 2007 179 577
- US-A1- 2007 255 377
- US-A1- 2007 299 493
- US-A1- 2007 299 493
- US-A1- 2008 288 040
- US-B1- 6 295 475
- US-B2- 6 606 522
- US-B2- 7 287 995
- US-B2- 7 363 090

## Description

The invention relates to a medical implantable lead of the kind being adapted to be implanted into a human or animal body for monitoring and/or controlling of an organ inside the body, comprising at a distal end a combined fixation means and electrode member in form of a helix, which is connected to a rotatable tubular torque transferring member being in its turn connected to a rotatable control member at a proximal end of the lead, and which is rotatable in relation to the lead and extendable out from the distal end, by rotation of the control member and the tubular torque transferring member, to be able to fixate the distal end of the lead to the organ by being screwed into the tissue, wherein the helix is electrically connected to a connector at the proximal end by means of at least one electrically conducting wire.

### Background of the invention

It is well known in the art to use a medical implantable lead of the above kind to monitor and/or control the function of an organ inside a human or animal body, for example to monitor and/or control a heart by means of a monitoring and/or controlling device in form of a pacemaker or cardiac defibrillator connected to the proximal end of the lead. The medical implantable lead is provided with at least one electrical conductor, in form of a coil having one or more helically formed electrically conducting wires, sometimes also referred to as filars, which connects one or more connectors arranged at the proximal end of the lead with one or more electrodes in its distal end. At least one of the electrodes is formed as a helix, which is adapted to be screwed into the tissue of the organ for receiving and/or transmitting electrical signals from and/or to the organ and transmit them, through the electrically conducting coil, to the monitoring and/or controlling device connected to the proximal end of the lead. The helix also functions as an attachment means for attaching the distal end of the lead to the organ by being rotatably extended out from the distal end of the lead and accordingly screwed into the tissue of the organ. To accomplish the rotation of the helix, it is mechanically connected to the innermost one of the electrically conducting coils, which accordingly has to be rotatable in relation to the lead as well as be sufficiently rigid to be able to transmit the required torque from the proximal to the distal end. The lead may also be provided with one or more additional electrodes separate from the helix and e.g. be formed as a contact electrode, abutting against a surface of the organ, or be formed as a so called indifferent electrode which is surrounded by body fluids such as blood.

Normally, such medical implantable leads are not considered to be compatible with Magnetic Resonance Imaging (MRI), i.e. persons or animals having such a lead implanted into the body, are excluded from being examined by MRI-scanning. This is due to the fact that the electromagnetic field, that is generated during the MRI-scanning, will induce a current in the conductor, which connects the one or more electrodes at the distal end of the medical implantable lead with the monitoring and/or controlling device at the proximal end of the lead. This induced current may cause heating at an electrode being in contact with the tissue of the organ, especially if the electrode is in form of a helix which is penetrated into and embedded within the tissue. If the heating is too high, there is a risk that this will cause damages to the tissue. However, the use of MRI-scanning for diagnostics is growing extensively and an increasing number of the population having a lead implanted would benefit from MRI-scans. It is thus desirable to reduce any heating at or close to the lead tip to acceptable and safe levels to allow MRI-scanning also of persons or animals having such a lead implanted.

It is known in the art to provide such medical implantable leads with an electrical shielding, in form of a tube of braided wires, which surrounds the coil and which in its proximal end normally is connected to the casing of the monitoring and/or controlling device. However, such shielded medical implantable leads are associated with several disadvantages. On the one hand, the braided shielding will give the medical implantable lead an increased thickness as well as increased rigidity, which normally is not desirable. On the other hand, it has appeared that such a braided shielding cannot prevent the induction of electrical current to the coiled conductor in a degree that is sufficient to, without risk, expose an individual, having an implanted lead, to a MRI-scanning.

US 7363090 discloses a way to reduce heating caused by induced current from MRI-scanning by connecting a contact electrode and an indifferent ring electrode in series with a band stop filter, which are tuned to certain frequencies utilized during MRI-scanning. Such a prior art medical implantable lead comprises passive electronic components, which contribute to making the lead more complex and thus more costly to manufacture.

US 2007/0299493 discloses a cardiac lead having a tubular torque-transmitting member extending through an interior lumen of the lead body. The tubular torque-transmitting member has a distal end connected to a rotatable fixation element and a proximal end connected to a rotatable actuator.

US 2004/0068299 discloses a medical system incorporating fluid delivery and lead delivery lumens dispense fluid into a volume of tissue. A hollow tissue piercing tip provides fluid delivery to the volume of tissue. The system includes a medical lead with an actie fixation member and a central lumen through which the tip is deployed.

### Summary of the invention

It is an object of the invention to provide a medical implantable lead, which in a simple and cost-effective way reduces the induction of current from an electromagnetic field into the electrically conducting coil. At least this object is achieved by a medical implantable lead according to claim 1.

The basis of the invention is the insight that the above object may be achieved by separating the function of effecting rotation and extending the helix out from the distal end of the lead, from the function of transmitting the electrical signals between the helix and, where appropriate, the one or more further electrodes at the distal end and the one or more connectors at the proximal end, i.e. to split these functions on separate members within the lead. More precisely, the function of transmitting a torque from the proximal end to the distal end for effecting rotation of and extending the helix out from the distal end, is effected by an inner tubular torque transferring member, which has no electrically conducting function to or from the electrode, whereas the electrically conducting function to and from the electrode is effected by a separate electrically conducting coil formed of one or more helical wires. Each wire is moreover coated with an electrically insulating layer, such that the coil will form an inductor, which will allow the low frequency signals between the electrode and the monitoring and/or controlling device to pass through without being exposed to high impedance. On the other hand, for induced current from high frequency electromagnetic fields, such as fields from MRI-scanning typically operating at 64 or 128 MHz, the impedance in the electrically conducting coil will be very high which to a large extent will restrain induced high frequency currents.

According to the invention, also at the proximal end of the lead, the function of performing rotation of the torque transferring member, and consequently the helix, is separated from the function of conducting an electrical current between a connector at the proximal end via the electrically conducting coil and the helix. More precisely, the rotation of the torque transferring member is effected by rotation of a control member at the proximal end, which control member is connected to the torque transferring member and rotatably arranged coaxially within a connector. The connector is in turn electrically connected to the electrically conducting coil, which is rotatably fixed in relation to the lead. In case the torque transferring member and/or the control member is electrically conductive, the connector and the control member are electrically insulated from each other to prevent induced current from a magnetic field into the torque transferring member and/or the control member to be transmitted to the connector and the electrically conducting coil, as well as the helix. In other words, the lead is arranged such that the electrical connection between the helix and the conducting wire is maintained irrespective of the rotational position of the helix, while no electrical connection is present between the helix and the tubular torque transferring member even though the helix is rotatable by means of the tubular torque transferring member, and the rotatable control member is rotatable within the connector.

By forming the medical implantable lead in this way, it is possible to form the tubular torque transferring member with a sufficient mechanical strength and stiffness to be able to transfer the torque required, from the proximal end to the distal end, to rotate the helix for extending it out from the distal end of the lead and screw it into the tissue. The electrically conducting coil, on the other hand, can be optimized to present an as high inductance as possible. For example, since the electrically conducting coil does not have to transfer any torque to the helix, it can be formed of one or only a few wires, which each can be made very thin. In this way, the pitch of the individual wires of the coil will be very low, which will increase the inductance of the coil. Also, since the electrically conducting coil will be positioned around the torque transferring member, the diameter of the coil can be made larger than what is possible with the prior art combined electrically conducting and torque transferring coils. This will also increase the inductance.

Within this overall idea, the invention may be modified in many different ways. As stated, the torque transferring member is tubular having an inner bore. This is done for the purpose of allowing insertion of a guide wire into the inner bore to enable guiding of the distal end of the lead to a desired location inside a body. Hence, the diameter of the inner bore is large enough for the guide wire to be inserted into the bore. Besides this requirement, the torque transferring member can be formed in many different ways. In the prior art, the torque transferring member is normally formed of three to five comparatively thick, metallic wires in order to function both as an electrical conductor as well as a torque transferring member. The several rather thick wires will give the coil a sufficient mechanical strength to transfer the required torque, but will also give the coil a rather large pitch, which will reduce the inductance. Also, the torque transferring member according to the invention may be formed in a similar way, with the exception that the wires are not electrically conducting between an electrode at the distal end and a connector at the proximal end. However, the torque transferring member could also be formed of an electrically non-conducting material, such as e.g. a polymeric material, which can be formed as a coil of one or more helical threads or as a flexible tubing.

The medical implantable lead can be provided with more than one electrode, e.g two electrodes for a bipolar lead, three electrodes for a tripolar lead, etc. The electrically conducting wires for each electrode can optionally be provided in separate coils, which are co-axially arranged in relation to each other, or two or more separate electrically conducting wires, dedicated for different electrodes, can be provided side by side in one and the same coil. One advantage with an embodiment according to the latter case is that the overall diameter of the lead can be made smaller than in the former case. However, the inductance will be somewhat lower in the latter case in relation to the former, since the pitch of each individual wire will be somewhat larger. This can be alleviated by forming the coil from wires having a sufficient thin cross section.

Since the helix functions as an electrode, and is rotatable in relation to the lead, together with the torque transmitting member, arrangements should be made in respect of the electrical connection of the helix to the electrically conducting coil. This is due to the fact that the electrically conducting coil dedicated for the helix is non-rotating while the torque transferring member is rotatable in relation to the electrically conducting coil. The electrical connection between the helix and the coil can be ensured by e.g. arranging a sliding electrical contact between the helix and the specific wire. However, the electrical connection between the electrically conducting coil and the helix may be maintained also in many other ways, as realized by the skilled person.

In a medical implantable lead according to the invention, the mechanical transfer of torque from the proximal to the distal end, for rotating and extending the helix for screwing it into the tissue, is separated from the electrical conducting between the monitoring and/or controlling device and an electrode member in form of a helix at the distal end. More precisely, the torque is transferred by means of a tubular torque transferring member, which does not transfer any electrical current between the monitoring and/or controlling device and the helix. On the other hand, the electrical signals are conducted by a coil arranged on the outside of the torque transferring member and formed by helically wound wires having an outer non-conducting layer, such that adjacent loops of the coil will be electrically insulated from each other and the coil will function as an inductor. By a medical implantable lead formed in this way, it is possible to achieve a sufficient inductance in the conducting coil to prevent or at least reduce the strength of induced high frequency current from an electromagnetic field, into the electrically conducting coil to a sufficient degree that is harmless for the tissue. At the same time, the torque transferring member can be made with a sufficient strength and rigidity to allow transferring of the torque required. In embodiments of the invention, the electrically conducting coil may e.g. be formed of a wire comprising a silver core, which presents an advantageous low resistance to the signals and therefore can be given a small cross sectional dimension, such that the coil can be formed with a small pitch which will increase the inductance. The non-conducting layer around the wire core may be formed of a mineral or a polymer, such as e.g. ETFE (Ethylene Tetra Fluor Ethylene).

Since the inner tubular torque transferring member is mechanically connected to the helix but has no electrically conducting function to and from the helix, whereas the electrically conducting coil arranged outside of the tubular torque transferring member is not adapted to mechanically transfer any torque to the helix, the one or more conducting wires in the electrically conducting coil are arranged to always maintain the electrical connection between a connector at the proximal end and the helix at the distal end irrespective of the rotated position of the tubular torque transferring member and the helix.

Within the overall idea, the invention may be altered and modified in many different ways. For example, the tubular torque transferring member may optionally be formed as a flexible tube or as a helical coil of one or more threads or wires. It may also optionally be formed of an electrically insulating or a conducting material. In the former case no special measures has to be taken for insulating the tubular torque transferring member electrically from the helix or from the connector at the proximal end, such as may have to be done in case the tubular torque transmitting member is formed of an electrically conducting material. The connecting structure at the proximal end of the lead, which is adapted to be connected to a monitoring and/or controlling device, is formed with a connector pin which comprises an inner control member and a connector arranged co-axially outside of the control member. Accordingly, at least a part of the outer surface of the connector pin will be formed by the connector, which is electrically connected to an electrically conducting coil and the helix and which is adapted to be electrically connected to the monitoring and/or controlling device. The control member is rotatably arranged within the connector and mechanically connected to the torque transferring member and the helix. The control member can optionally be entirely surrounded by the connector such that only a proximal end of the control member is visible and accessible, in which case some form of engagement means has to be formed at the end surface for engagement with a suitable rotary tool for performing rotation of the torque transferring member and the helix, or project a distance out from the proximal end of the connector such that it forms a part of the surface of the connector pin. In the latter case rotation may be performed by gripping the control member by means of a gripping tool around the outer surface. Preferably, the projecting part of the control member is formed with an enlarged cross section such that it will have the same diameter as the connector. Moreover, the control member is preferably electrically insulated from the connector in case the torque transferring member and/or the control member is electrically conductive, e.g. metallic. The electrical insulation can preferably be formed as a sleeve surrounding the control member, but could also be formed as e.g. two ring members at a distance from each other. The control member may either be rotatable within the electrical insulation, or the electrical insulation be rotatable within the connector.

The embodiments described and illustrated hereinafter are given solely for exemplifying reasons and are not intended to be comprehensive. Accordingly, many other embodiments could be conceivable within the scope of the invention.

### Brief description of the drawings

The invention will hereinafter be described by way of example by reference to embodiments illustrated in the accompanying drawings, in which:
- Fig 1: is a perspective view of a medical implantable lead;
- Fig 2: is a view in an enlarged scale of the lead in fig 1 in a shortened state showing only the proximal and the distal ends of the lead;
- Fig 3: is a longitudinal section along the line A-A in fig 2 of a portion of a medical implantable lead according to a first embodiment;
- Fig 4: is a cross section along the line B-B in fig 2 of the lead according to fig 3;
- Fig 5: is a longitudinal section along the line A-A in fig 2 of a portion of a medical implantable lead according to a second embodiment;
- Fig 6: is a cross section along the line B-B in fig 2 of the lead according to fig 5;
- Fig 7: is a longitudinal section through a distal portion of the medical implantable lead, illustrating an embodiment of the electrically connection to the electrodes as well as the mechanically connection to the helix, which is in a retracted state;
- Fig 8: is a longitudinal section according to fig 7 with the helix in an extended state;
- Fig 9: is a longitudinal section through the proximal end of a lead according to a first embodiment of the invention;
- Fig 10: is a combined longitudinal section and perspective view of the lead according to fig 9 together with a perspective view of a rotary tool;
- Fig 11: is a perspective view of the proximal end of the lead according to figs 9 and 10;
- Fig 12: is a perspective view of a rotary tool for interaction with a medical implantable lead according to figs 9-11,
- Fig 13: is a longitudinal section through the proximal end of a lead according to a second embodiment of the invention ;
- Fig 14: is a combined longitudinal section and perspective view of the lead according to fig 13;
- Fig 15: is a perspective view of the proximal end of the lead according to figs 13 and 14; and
- Fig 16: is a perspective view of the proximal end of the lead and a clamp gripping around the control member.

### Detailed description of embodiments of the invention

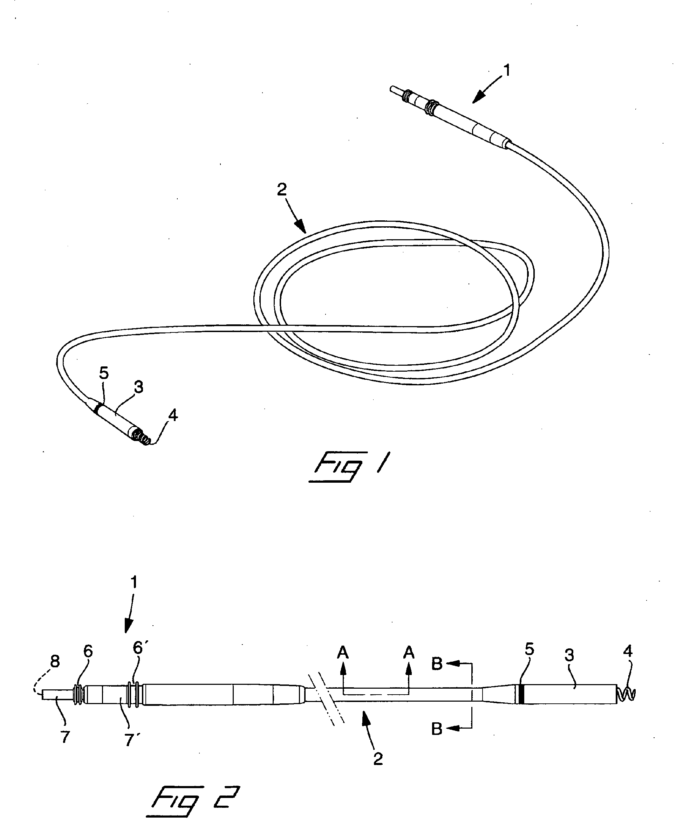

Reference is first made to fig 1, in which is illustrated a medical implantable lead according to the invention in a perspective view. The lead comprises a connecting structure 1 at a proximal end for connection to a not shown monitoring and/or controlling device such as a pacemaker or the like, an intermediate flexible lead part 2, and a so called header 3 at a distal end. The header is provided with a helix 4, which can be screwed out in the axial direction of the lead from a cavity at the distal end of the header. The helix has the function of attaching the distal end of the lead to the heart, by being screwed into the tissue, and also functions as an electrode for receiving and/or transmitting electrical signals from and to the tissue, respectively. The header is also provided with a second electrode, a so called indifferent electrode 5, which is formed as a ring and positioned a small distance from the distal end and has the purpose of forming a complete current path together with the helix.

The proximal and the distal ends of the lead according to fig 1, are illustrated in an enlarged scale in the shortened representation of the lead in fig 2. The helix 4 for fixation of the distal end of the lead to tissue as well as for function as an electrode is shown in an extended state. However, during insertion of the lead into a body, the helix is retracted into the bore of the header 3 having a tubular shape at the distal end. In addition to a tip electrode in form of the helix, which is adapted to be screwed into the tissue, the lead comprises, as is mentioned above, a second electrode in form of the ring electrode 5 on a short distance from the distal end.

At the proximal end, the connecting structure 1 for connection to a not shown monitoring and/or controlling device comprises a first fluid tight sealing member 6 and a second fluid tight sealing member 6'. The sealing members are formed of an elastic material, in order to achieve a fluid tight connection to a socket recess of the monitoring and/or controlling device. In the area being positioned proximal in relation to the first sealing member 6, the lead is provided with a first electrically conducting connector 7 and in the area between the first and second sealing members the lead is provided with a second electrically conducting connector 7', as is described more in detail below, which are adapted to be electrically coupled to mating connectors inside the monitoring and/or controlling device. The first connector 7 is in electrical contact with the helix 4, whereas the second connector 7' is in electrical contact with the ring electrode 5 by means of one or more electrical conducting coils inside the lead, as is to be explained more in detail below. In the most proximal end, the lead is provided with a rotatable control member 8, which in the embodiment of fig 2 is surrounded by the first connector 7 and accordingly not visible therein. The control member 8 is, according to the invention, separated from the connectors 7, 7' and by means of the control member the helix 4 can be rotated and screwed out from the bore inside the header 3 and into the tissue.

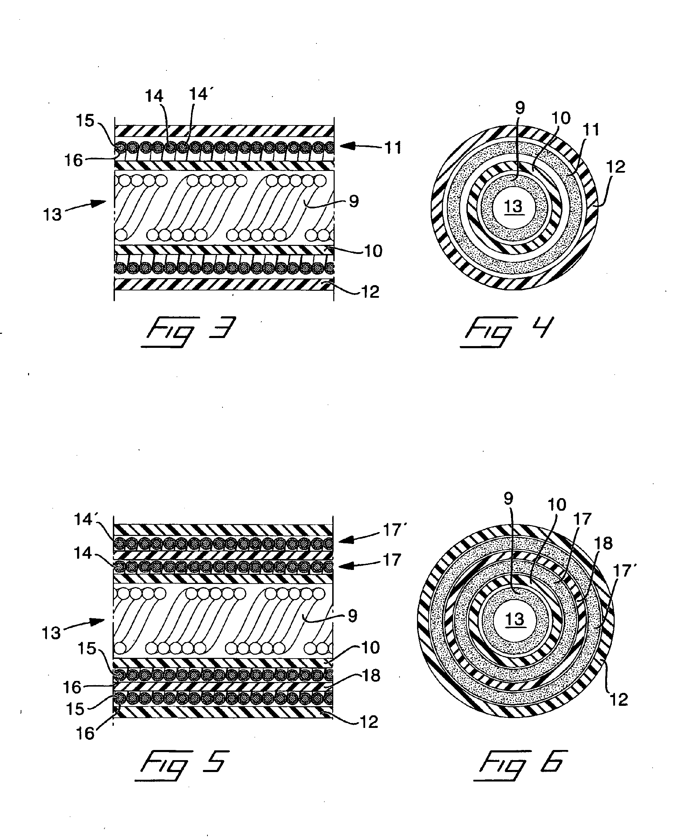

Now reference is made to figs 3 and 4, in which are illustrated a first embodiment of the flexible lead part 2 in a longitudinal section as well as a cross section through the lead, respectively. The lead comprises an inner tubular torque transferring member 9, an inner fluid tight tubing 10, an electrically conducting coil 11 and an outer fluid tight tubing 12. The inner tubular torque transferring member is rotatable arranged inside the inner tubing and is formed as a coil of five comparatively thick and rigid helical wires of e.g. metal or polymer, such that it is well suited for transferring of a torque from the proximal to the distal end of the lead. Moreover, the torque transferring member 9 defines an inner bore 13 for the purpose of allowing insertion of a guide wire or the like for guiding the tip of the lead to a desired position inside a body. The electrically conducting coil 11 is composed of two separate, coradially wound wires 14, 14', each having an electrically conducting core 15 and a surrounding electrically insulating layer 16, such that they form two electrically separated inductance coils.

With reference also to fig 2, it is to be understood that the structure of the flexible lead part 2 as illustrated in figs 3 and 4, extends from the connecting structure 1 at the proximal end to the header 3 at the distal end. Moreover, the tubular torque transferring member 9 is in its proximal end mechanically connected to the rotatable control member 8 and in its distal end mechanically connected to the helix 4, such that by rotating the rotatable control member it is possible to rotate the helix and extend it out from the inner bore of the header and screw it into the tissue. One of the wires in the electrically conducting coil 11 is in its proximal end electrically connected to the first connector 7 and in its distal end electrically connected to the helix 4, whereas the other wire in the electrically conducting coil is in its proximal end electrically connected to the second connector 7' and in its distal end electrically connected to the ring electrode 5.

Reference is then made to figs 5 and 6, in which are illustrated a second embodiment of the flexible lead part 2 in a longitudinal section as well as a cross section through the lead, respectively. As in the first embodiment according to figs 3 and 4, this embodiment comprises an inner tubular torque transferring member 9, formed in a similar way as in the first embodiment of five helical wires, and an inner fluid tight tubing 10. However, this embodiment comprises two separate electrically conducting coils, one inner coil 17 and one outer coil 17', separated by an intermediate fluid tight tubing 18. Each of the electrically conducting coils is formed of one single wire 14 and 14', respectively, having an electrically conducting core 15 and a surrounding electrically insulating layer 16, such that they form two coaxially arranged inductance coils. Also this embodiment comprises an outer fluid tight tubing 12.

As in the first embodiment, the tubular torque transferring member 9 is in its proximal end mechanically connected to the rotatable control member 8 and in its distal end mechanically connected to the helix 4, such that by rotating the control member it is possible to rotate the helix and extend it out from the inner bore of the header and screw it into the tissue. The inner electrically conducting coil 17 is in its proximal end electrically connected to the first connector 7 and in its distal end electrically connected to the helix 4, whereas the outer electrically conducting coil 17' is in its proximal end electrically connected to the second connector 7' and in its distal end electrically connected to the ring electrode 5.

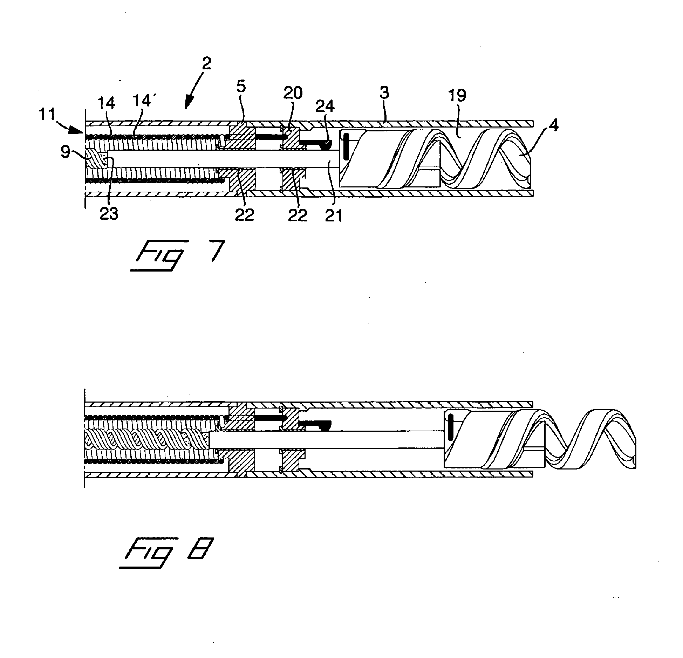

Reference is then made to figs 7 and 8 of the drawings, in which is illustrated an embodiment of a connection of the electrically conducting wires 14,14' to the electrodes as well as the tubular torque transferring member 9 to the helix 4. Figs 7 and 8 are longitudinal sections through the distal portion of a medical implantable lead, according to the embodiment as illustrated and described in relation to figs 3 and 4, with the helix being retracted and extended, respectively.

The longitudinal sections of figs 7 and 8 are taken at the joint between the header 3, as seen to the right, and the distal end portion of the flexible lead part 2 as illustrated in figs 3 and 4. The header is made of a rigid material such as metal or a polymer and is formed with an inner bore 19, in which the helix 4 is rotatably and displaceably accommodated. In the joint region between the header and the flexible lead part, the electrically conducting ring electrode 5 is provided, which also functions as a joint connector in that it comprises a distal shoulder surface, in which the proximal end of the header 3 is located and attached, and a proximal shoulder surface in which the distal end of the flexible lead part 2 is located and attached. A short distance towards the distal end from the ring electrode 5, the lead is provided with a fixed support member 20. Both the ring electrode 5 and the support member 20 is formed with a through bore, through which a shaft 21 is rotatably and displaceably inserted, at the distal end of which the helix 4 is mounted. The shaft 21 is of an electrically conducting material and to prevent electrical connection between the shaft 21 and the ring electrode 5 as well as the support member 20, in case it is manufactured of an electrically conducting material, electrically insulating shaft bushings 22 are arranged in each of the through bores. To allow rotation and displacing of the helix 4 out from and into the inner bore 19 of the header, the tubular torque transmitting member is mechanically connected to the proximal end of the shaft. In case the tubular torque transferring member 9 is of an electrically conducting material, the connection is arranged in an electrically non-conducting fashion, such as via an electrically insulating sleeve 23 or the like. The electrically conducting coil of the lead comprises two electrically conducting wires 14, 14', which are electrically insulated from each other. To accomplish electrical connection to each of the ring electrode 5 and the helix 4, one of the electrical conducting wires 14' is electrically connected to the ring electrode 5, whereas the other electrically conducting wire 14 is electrically connected to a sliding contact 24 arranged on the support member 20, the sliding contact being in permanent electrically contact with the shaft 21, which in its turn is in electrically contact with the helix 4. In this way an electrically connection is ensured with the helix in spite of the fact that the tubular torque transferring member 9 is not electrically conducting and irrespective of the position of the helix.

The embodiment of figs 7 and 8 are only an exemplifying embodiment of how the mechanical and electrical connections between the helix 4 and the tubular torque transferring member 9 and the electrically conducting coils 14,14', respectively, can be maintained as well as separated. It is to be understood, however, that this embodiment is only exemplary and that these functions can be realized also in many other different ways.

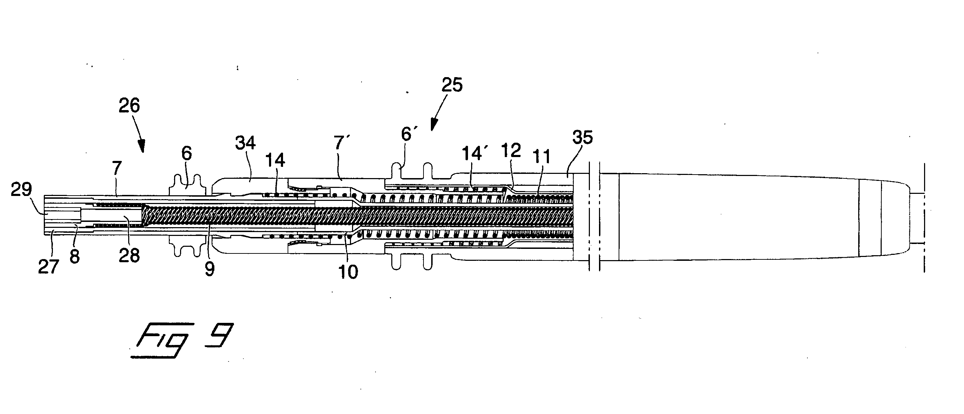

Now reference is made to figs 9 to 11 for a detailed description of a first embodiment of the arrangement of the connecting structure at the proximal end of the medical implantable lead according to the invention. As can be seen, the connecting structure is comprised of a thickened portion 25 and a connector pin 26 protruding from the proximal end of the thickened portion. A first fluid tight sealing member 6 is arranged around the connector pin adjacent the proximal end of the thickened portion. A second fluid tight sealing member 6' is arranged around the thickened portion in an intermediate position of the same.

In prior art, normally the entire connector pin 26 is metallic and functions both as an electrical connector, which is in electrical connection with an inner electrically conducting coil connected to the helix at the distal end, as well as a rotatable control member for performing rotation of the helix by being rotatably mounted in the thickened portion. However, according to the invention, the connector pin is composed of an inner rotatable control member 8, which is connected to the inner torque transferring member 9 and is rotatably arranged within an outer, tubular electrically conducting connector 7, which in its turn is unrotatably mounted to the thickened portion and electrically connected to the helix 4 at the distal end via an electrically conducting coil 11, which is separated from the torque transferring member 9.

In this embodiment, the torque transferring member 9 and the control member 8 are metallic and accordingly electrically conductive. To prevent transfer of any electrical current, which may be induced into the torque transferring member by a surrounding electromagnetic field, from the torque transferring member to the electrically conducting coil 11 and hence to the helix 4, there is arranged an electrically insulating layer in form of an insulating tube 27 between the control member 8 and the connector 7. The insulating tube may optionally be unrotatably mounted to the connector 7, in which case the control member 8 is rotatable inside the insulating tube, or be unrotatably mounted to the control member, in which case the control member and the insulating tube are jointly rotatable within the connector. In case the control member and/or the torque transferring member would be of an electrically non-conductive material, the insulating tube could be dispensed with, since in that case no current can be induced into the torque transferring member from an electromagnetic field.

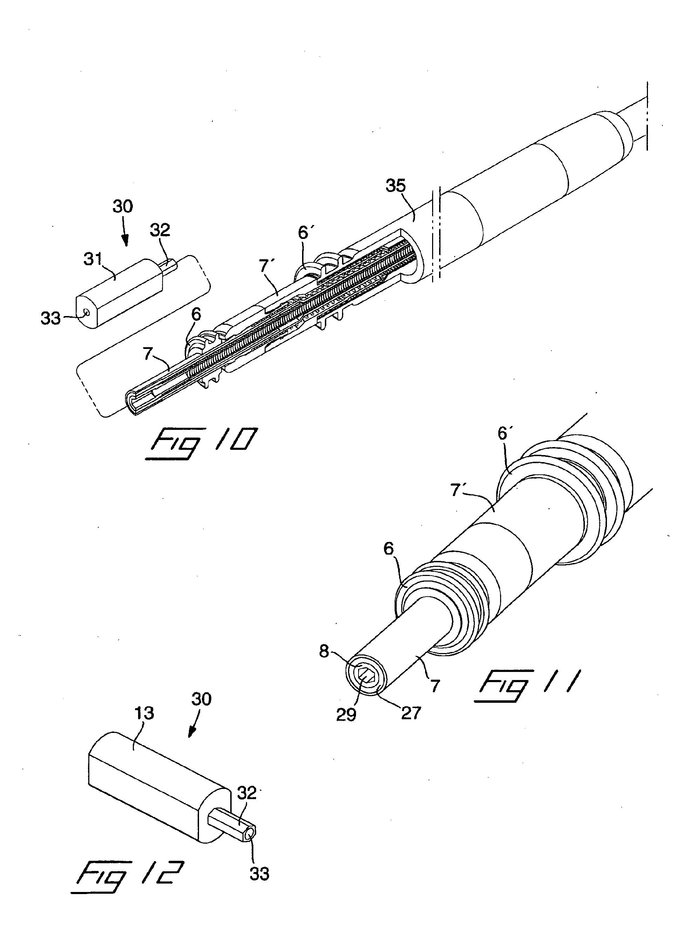

The control member 8 is tubular with a through bore 28 to allow insertion of a not shown guide wire through the control member and the tubular torque transferring member to the distal end of the lead. This is done for the purpose of performing guiding of the distal end of the lead, by means of the guide wire, to a desired position within a body, e.g. within a heart. As is evident from the drawings, in this embodiment the control member 8 is located entirely within the connector 7. To allow rotation of the control member, and hence also the torque transferring member and the helix, the proximal end of the through bore 28 in the control member is hexagonally formed to provide an engagement means 29 for a complementary formed rotary tool 30, as is illustrated in figs 10 and 12. The rotary tool is formed with a body 31 and a protruding shaft 32 having a hexagonal cross section to be inserted into the hexagonally formed engagement means 29 in the control member 8. Also the rotary tool 30 is provided with a through bore 33 through the body and the shaft to allow insertion of the guide wire while the rotary tool is connected to the lead.

The thickened portion of the connecting structure comprises a proximal electrically insulating portion 34, an intermediary electrically conducting second connector 7' and a distal electrically insulating sleeve 35. The lead according to this embodiment is provided with only one electrically conducting coil 11. However, the electrically conducting coil comprises two separate electrically conducting wires 14,14', each surrounded by an electrically insulating layer 16, as is illustrated in figs 3 and 4. One of the wires 14 is electrically connected to the helix 4 at the distal end and to the distal end of the first connector 7 within the proximal electrically insulated portion 34 of the thickened portion of the connecting structure at the proximal end of the lead. The other electrically conducting wire 14' of the electrically conducting coil 11 is connected to the indifferent electrode 5 at the distal end and to a distal end of the second connector 7' which is formed as a protruding flange having a smaller cross sectional dimension than the rest of the connector. The connections between the first and second electrically conducting wires of the electrically conducting coil and the first and second connector, respectively, appears from figs 9 and 10.

As is also seen from figs 9 and 10, the outer fluid tight tubing 12 of the electrically conducting coil 11 is thread over the flange portion of the second connector 7' and the outer flexible sleeve 35 is thread onto the flange portion over the fluid tight tubing for protecting the transition section between the connecting structure and the flexible lead part.

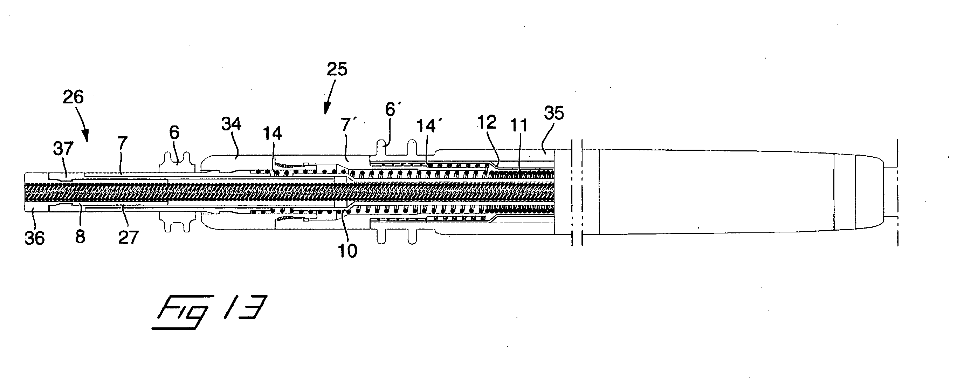

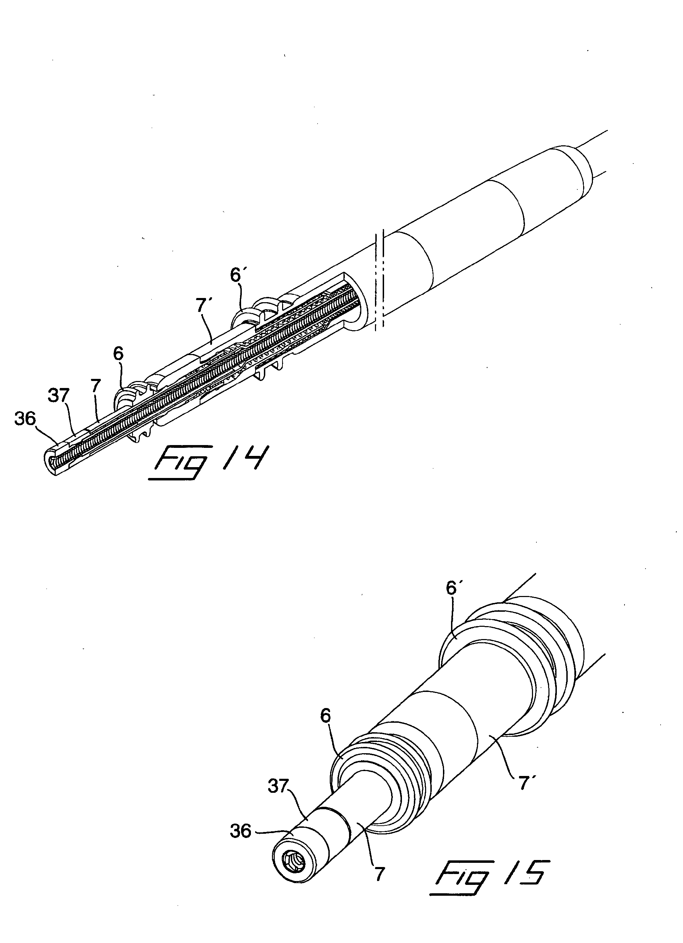

Reference is then made to figs 13-15 in which is illustrated a second embodiment of the arrangement of the connecting structure at the proximal end of the medical implantable lead according to the invention.

As in the embodiment according to figs 9-11, the connecting structure according to this embodiment comprises a thickened portion 25 and a connector pin 26 protruding from the proximal end of the thickened portion. A first fluid tight sealing member 6 is arranged around the connector pin adjacent the proximal end of the thickened portion. A second fluid tight sealing member 6' is arranged around the thickened portion in an intermediate position of the same.

Moreover, the connector pin 26 is composed of an inner rotatable control member 8, which is connected to the inner torque transferring member 9 and is rotatably arranged within an outer, tubular electrically conducting connector 7, which in its turn is unrotatably mounted to the thickened portion and electrically connected to the helix 4 at the distal end via an electrically conducting coil 11, which is separated from the torque transferring member 9.

The torque transferring member 9 and the control member 8 are metallic and accordingly electrically conductive. To prevent transfer of any electrical current from the torque transferring member to the electrically conducting coil 11 and hence to the helix 4, there is arranged an electrically insulating layer in form of an insulating tube 27 between the control member 8 and the connector 7. The insulating tube may optionally be unrotatably mounted to the connector 7, in which case the control member 8 is rotatable inside the insulating tube, or be unrotatably mounted to the control member, in which case the control member and the insulating tube are jointly rotatable within the connector.

The control member 8 is tubular with a through bore 28 to allow insertion of a not shown guide wire through the control member and the tubular torque transferring member to the distal end of the lead. This is done for the purpose of performing guiding of the distal end of the lead, by means of the guide wire, to a desired position within a body, e.g. within a heart.

The thickened portion of the connecting structure comprises a proximal electrically insulating portion 34, an intermediary electrically conducting second connector 7' and a distal electrically insulating sleeve 35. The lead also according to this embodiment is provided with only one electrically conducting coil 11, comprising two separate electrically conducting wires 14, 14', each surrounded by an electrically insulating layer 16, as is illustrated in figs 3 and 4. One of the wires 14 is electrically connected to the helix 4 at the distal end and to the distal end of the first connector 7 within the proximal electrically insulated portion 34 of the thickened portion of the connecting structure at the proximal end of the lead. The other electrically conducting wire 14' of the electrically conducting coil 11 is connected to the indifferent electrode 5 at the distal end and to a distal end of the second connector 7' which is formed as a protruding flange having a smaller cross sectional dimension than the rest of the connector. The connections between the first and second electrically conducting wires of the electrically conducting coil and the first and second connector, respectively, appears from figs 9 and 10.

As is also seen from figs 9 and 10, the outer fluid tight tubing 12 of the electrically conducting coil 11 is thread over the flange portion of the second connector 7' and the outer flexible sleeve 35 is thread onto the flange portion over the fluid tight tubing for protecting the transition section between the connecting structure and the flexible lead part.

As described so far, this embodiment is identical with the first embodiment. However, in this embodiment the connector pin 26 has a somewhat different structure. More precisely, the control member 8 is not entirely surrounded by the connector 7. Instead the control member projects from the proximal end of the connector where it is formed with an increased proximal portion 36 having a cross sectional dimension that is equal to the cross sectional dimension of the rest of the connector pin and consequently the control member constitutes the outer surface of the proximal end of the connector pin. To insulate the control member 8 electrically from the first connector 7, also the insulating tube 27 between the control member and the connector is formed with an increased proximal portion 37 having a cross sectional dimension that is equal to the cross sectional dimension of the rest of the connector pin, such that the connector pin is provided with an insulating surface between the control member and the first connector.

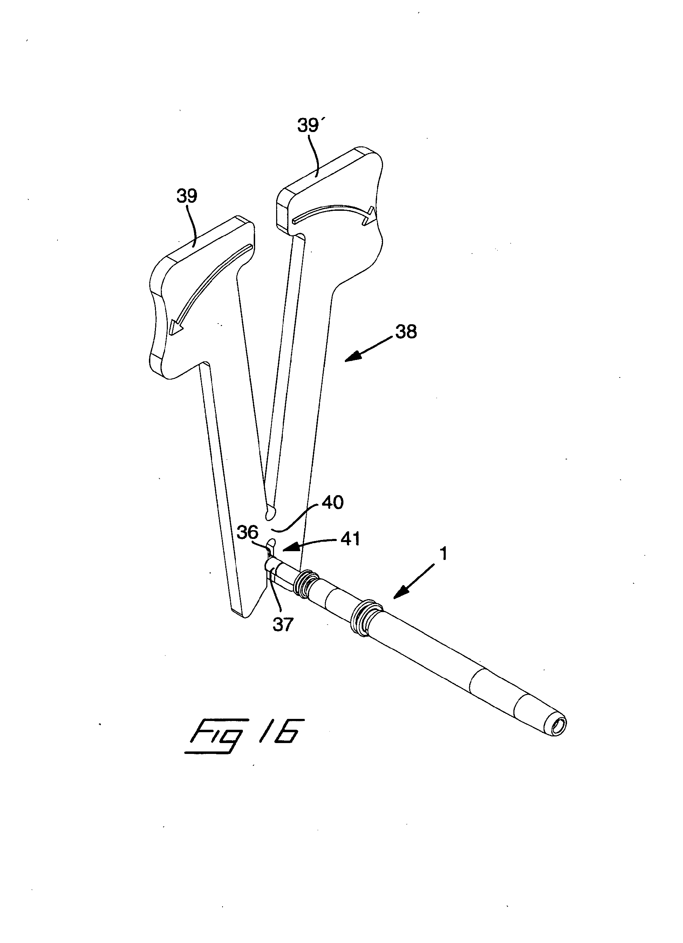

Moreover, the control member is not formed with any specific engagement means for engagement with a rotary tool, as in the embodiment according to figs 9-11. To rotate the torque transferring member and the helix during implanting of the lead inside a body, the proximal portion 36 may instead be gripped by means of an arbitrary gripping tool such as a clamp 38, as is illustrated in fig 16. The clamp 38 is made of an elastic material, such as a plastic, and comprises two shanks 39, 39' which are formed as one unitary piece and being connected in a connecting portion 40 adjacent a lower end, wherein the clamp forms a gripping portion 41 in form of a recess below the connecting portion. Accordingly, by gripping upper ends of the shanks 39, 39' by hand, a physician can displace them towards each other, in which case the gripping portion 41 in the lower end will open up due to elastic deformation in the connecting portion 40 such that the gripping portion may be positioned over the proximal portion 36 of the control member 8. Due to the elastic characteristics in the material, the gripping portion will clamp around the control member such that the physician may rotate the control member, and hence also the helix 4 at the distal end, by rotating the clamp 38.

## Claims

1. An MRI-compatible medical implantable lead of the kind being adapted to be implanted into a human or animal body for monitoring and/or controlling an organ inside the body, comprising:
a connecting structure (1) comprising a connector pin (26) at a proximal end of the lead, said connector pin (26) comprising a rotatable control member (8);
a rotatable tubular torque transferring member (9) connected to the rotatable control member (8);
a combined fixation means and electrode member in form of a helix (4) at a distal end of the lead, which helix (4) is connected to said rotatable tubular torque transferring member (9) and is rotatable in relation to the lead and extendable out from said distal end, by rotation of the control member and the tubular torque transferring member, for fixation of the lead to the organ,
a connector (7) at said proximal end of the lead electrically connected to the helix by means of at least one electrically conducting wire (14) formed as an electrically conducting coil (11), which is separate from the tubular torque transferring member (9) and rotatably fixed in relation to the lead, whereby the tubular torque transferring member (9) is rotatably arranged within the electrically conducting coil and has no electrically conducting function to or from the helix (4),
**characterized in that** the electrically conducting coil (11) comprises one or more individual wires, each comprising an electrically conducting wire core (15) and a surrounding electrically insulating layer (16), and
that said connector pin (26) comprises said control member (8) and said connector (7), whereby said control member (8) is rotatably arranged within said connector (7).

2. A medical implantable lead according to claim 1, **characterized in that** the control member (8) is electrically insulated from the connector (7).

3. A medical implantable lead according to claim 2, **characterized in that** the control member (8) and the connector (7) are electrically insulated from each other by means of an electrically insulating member (27).

4. A medical implantable lead according to claim 3, **characterized in that** the electrically insulating member is an electrically insulating sleeve (27) and the connector (7) is arranged at the outer circumference of the insulating sleeve.

5. A medical implantable lead according to claim 3 or 4, **characterized in that** the electrically insulating member (27) is in one unitary piece.

6. A medical implantable lead according to any of claims 3-5, **characterized in that** the electrically insulating member (27) is unrotatably mounted to said connector (7), whereby said control member (8) is rotatable inside said insulating member (27).

7. A medical implantable lead according to any of claims 3-5, **characterized in that** the electrically insulating member (27) is unrotatably mounted to said control member (8), whereby said control member (8) and said electrically insulating member (27) are jointly rotatable within said connector (7).

8. A medical implantable lead according to any of the preceding claims, **characteri**- z e d in that the connector (7) is a metallic sleeve completely surrounding the control member (8).

9. A medical implantable lead according to claim 8, **characterized in that** the control member (8) is formed with a rotary engagement portion (29) for a rotary tool (30).

10. A medical implantable lead according to claim 9, **characterized in that** the rotary engagement portion is in form of a recess having engagement formations (29) at the proximal end of the control member (8).

11. A medical implantable lead according to any of the claims 1-7, **characterized in that** the control member (8) projects with a proximal portion (36) beyond the connector (7) at the proximal end of the lead.

12. A medical implantable lead according to claim 11, **characterized in that** the proximal portion (36) of the control member (8) is adapted to be gripped by a gripping tool (38) for rotation of the control member (8).

13. A medical implantable lead according to any of the preceding claims, **characteri**- z e d in that said connecting structure (1) further comprises a thickened portion (25) and that said connector (7) is unrotatably mounted to said thickened portion (25).

## Patentansprüche

1. Ein MRT-kompatibler, medizinisch implantierbarer Schlauch, derart ausgelegt, in einen menschlichen oder einen tierischen Körper implantiert zu werden, zur Überwachung und/oder zur Kontrolle eines Organs im Inneren des Körpers, umfassend:
eine Verbindungsstruktur (1), die einen Steckerstift (26) an einem proximalen Ende des Schlauchs umfasst, wobei der Steckerstift (26) ein drehbares Kontrollglied (8) umfasst;
ein drehbares, rohrförmiges Drehmoment-übertragendes Glied (9), das mit dem drehbaren Kontrollglied (8) verbunden ist;
ein kombiniertes Befestigungsmittel und Elektrodenmittel in Form einer Helix (4) an einem distalen Ende des Schlauchs, wobei die Helix (4) mit dem drehbaren rohrförmigen Drehmoment-übertragenden Glied (9) verbunden ist und relativ zu dem Schlauch drehbar ist und aus dem distalen Ende ausziehbar ist, durch Rotation des Kontrollglieds und des rohrförmigen Drehmoment-übertragenden Glieds, zur Fixierung des Schlauchs an dem Organ,
einen Konnektor (7) an dem proximalen Ende des Schlauchs, der mit der Helix elektrisch durch wenigstens einen elektrisch leitenden Draht (14) verbunden ist, der als eine elektrisch leitende Spule (11) ausgebildet ist, die von dem rohrförmigen Drehmoment-übertragenden Glied (9) getrennt ist, und die in Relation zu dem Schlauch drehbar angebracht ist, wobei das rohrförmige Drehmomentübertragende Glied (9) innerhalb der elektrisch leitenden Spule drehbar angeordnet ist und keine elektrisch leitende Funktion zu oder von der Helix (4) aufweist,
**dadurch gekennzeichnet, dass** die elektrisch leitende Spule (11) einen oder mehrere einzelne Drähte umfasst, wobei jeder einen elektrisch leitenden Drahtkern (15) und eine umgebende, elektrisch isolierende Schicht (16) umfasst, und
dass der Steckerstift (26) das Kontrollglied (8) und den Konnektor (7) umfasst, wobei das Kontrollglied (8) drehbar innerhalb des Konnektors (7) angeordnet ist.

2. Medizinisch implantierbarer Schlauch nach Anspruch 1, **dadurch gekennzeichnet, dass** das Kontrollglied (8) gegenüber dem Konnektor (7) elektrisch isoliert ist.

3. Medizinisch implantierbarer Schlauch nach Anspruch 2, **dadurch gekennzeichnet, dass** das Kontrollglied (8) und der Konnektor (7) gegeneinander elektrisch durch ein elektrisch isolierendes Glied (27) isoliert sind.

4. Medizinisch implantierbarer Schlauch nach Anspruch 3, **dadurch gekennzeichnet, dass** das elektrisch isolierende Glied eine elektrisch isolierende Hülse (27) ist, und dass der Konnektor (7) an dem Außenumfang der isolierenden Hülse angeordnet ist.

5. Medizinisch implantierbarer Schlauch nach Anspruch 3 oder 4, **dadurch gekennzeichnet, dass** das elektrisch isolierende Glied (27) einstückig ausgebildet ist.

6. Medizinisch implantierbarer Schlauch nach einem der Ansprüche 3 bis 5, **dadurch gekennzeichnet, dass** das elektrisch isolierende Glied (27) an dem Konnektor (7) undrehbar befestigt ist, wobei das Kontrollglied (8) im Inneren des isolierenden Glieds (27) drehbar angeordnet ist.

7. Medizinisch implantierbarer Schlauch nach einem der Ansprüche 3 bis 5, **dadurch gekennzeichnet, dass** das elektrisch isolierende Glied (27) an dem Kontrollglied (8) undrehbar befestigt ist, wobei das Kontrollglied (8) und das elektrisch isolierende Glied (27) gemeinsam innerhalb des Konnektors (7) drehbar sind.

8. Medizinisch implantierbarer Schlauch nach einem der vorhergehenden Ansprüche, **dadurch gekennzeichnet, dass** der Konnektor (7) eine metallische Hülse ist, die das Kontrollglied (8) vollständig umgibt.

9. Medizinisch implantierbarer Schlauch nach Anspruch 8, **dadurch gekennzeichnet, dass** das Kontrollglied (8) mit einem drehbaren Eingriffsteil (29) für ein drehbares Werkzeug (30) ausgebildet ist.

10. Medizinisch implantierbarer Schlauch nach Anspruch 9, **dadurch gekennzeichnet, dass** das drehbare Eingriffsteil in Form einer Aussparung ausgebildet ist, die an dem proximalen Ende des Kontrollglieds (8) Eingriffsformationen (29) aufweist.

11. Medizinisch implantierbarer Schlauch nach einem der Ansprüche 1 bis 7, **dadurch gekennzeichnet, dass** das Kontrollglied (8) mit einem proximalen Teil (36) an dem proximalen Ende des Schlauchs über den Konnektor (7) hinausragt.

12. Medizinisch implantierbarer Schlauch nach Anspruch 11, **dadurch gekennzeichnet, dass** der proximale Teil (36) des Kontrollglieds (8) ausgelegt ist, zur Drehung des Kontrollglieds (8) von einem Greifwerkzeug (38) gegriffen zu werden.

13. Medizinisch implantierbarer Schlauch nach einem der vorhergehenden Ansprüche, **dadurch gekennzeichnet, dass** die Verbindungsstruktur (1) weiter einen verdickten Bereich (25) umfasst, und dass der Konnektor (7) undrehbar an dem verdickten Bereich (25) befestigt ist..

## Revendications

1. Sonde médicale implantable compatible avec l'IRM du type apte à être implantée dans un corps humain ou animal pour surveiller et/ou contrôler un organe à l'intérieur du corps, comprenant :
une structure de connexion (1) comprenant une broche de connexion (26) à une extrémité proximale de la sonde, ladite broche de connexion (26) comprenant un élément de commande rotatif (8) ;
un élément tubulaire de transfert de couple rotatif (9) raccordé à l'élément de commande rotatif (8) ;
un élément combiné associant moyen de fixation et électrode sous la forme d'une hélice (4) à une extrémité distale de la sonde, laquelle hélice (4) est raccordée audit élément tubulaire de transfert de couple rotatif (9) et est rotative par rapport à la sonde et peut s'étendre hors de ladite extrémité distale, par rotation de l'élément de commande et de l'élément tubulaire de transfert de couple, pour la fixation de la sonde sur l'organe,
un connecteur (7) à ladite extrémité proximale de la sonde, connecté électriquement à l'hélice au moyen d'au moins un fil électroconducteur (14) agencé sous la forme d'une bobine électroconductrice (11), laquelle est séparée de l'élément tubulaire de transfert de couple (9) et montée rotative par rapport à la sonde, moyennant quoi l'élément tubulaire de transfert de couple (9) est agencé rotatif à l'intérieur de la bobine électroconductrice et n'a aucune fonction électroconductrice vis-à-vis de l'hélice (4),
**caractérisée en ce que** la bobine électroconductrice (11) comprend un ou plusieurs fils individuels comprenant chacun une âme de fil électroconducteur (15) et une couche diélectrique (16) qui l'enveloppe, et
**en ce que** ladite broche de connexion (26) comprend ledit élément de commande (8) et ledit connecteur (7), moyennant quoi ledit élément de commande (8) est agencé rotatif à l'intérieur dudit connecteur (7).

2. Sonde médicale implantable selon la revendication 1, **caractérisée en ce que** l'élément de commande (8) est électriquement isolé vis-à-vis du connecteur (7).

3. Sonde médicale implantable selon la revendication 2, **caractérisée en ce que** l'élément de commande (8) et le connecteur (7) sont électriquement isolés l'un vis-à-vis de l'autre au moyen d'un élément diélectrique (27).

4. Sonde médicale implantable selon la revendication 3, **caractérisée en ce que** l'élément diélectrique est un manchon diélectrique (27) et le connecteur (7) est agencé sur la circonférence extérieure du manchon diélectrique.

5. Sonde médicale implantable selon la revendication 3 ou 4, **caractérisée en ce que** l'élément diélectrique (27) se trouve sous la forme d'une pièce unitaire.

6. Sonde médicale implantable selon l'une quelconque des revendications 3 à 5, **caractérisée en ce que** l'élément diélectrique (27) est monté non rotatif sur ledit connecteur (7), moyennant quoi ledit élément de commande (8) est rotatif à l'intérieur dudit élément diélectrique (27).

7. Sonde médicale implantable selon l'une quelconque des revendications 3 à 5, **caractérisée en ce que** l'élément diélectrique (27) est monté non rotatif sur ledit élément de commande (8), moyennant quoi ledit élément de commande (8) et ledit élément diélectrique (27) sont solidaires en rotation à l'intérieur dudit connecteur (7).

8. Sonde médicale implantable selon l'une quelconque des revendications précédentes, **caractérisée en ce que** le connecteur (7) est un manchon métallique enveloppant complètement l'élément de commande (8).

9. Sonde médicale implantable selon la revendication 8, **caractérisée en ce que** l'élément de commande (8) présente une partie d'engagement rotative (29) destinée à un outil rotatif (30).

10. Sonde médicale implantable selon la revendication 9, **caractérisée en ce que** la partie d'engagement rotative se trouve sous la forme d'un creux présentant des formations d'engagement (29) à l'extrémité proximale de l'élément de commande (8).

11. Sonde médicale implantable selon l'une quelconque des revendications 1 à 7, **caractérisée en ce que** l'élément de commande (8) fait saillie par une partie proximale (36) au-delà du connecteur (7) à l'extrémité proximale de la sonde.

12. Sonde médicale implantable selon la revendication 11, **caractérisée en ce que** la partie proximale (36) de l'élément de commande (8) est adaptée pour être saisie par un outil de saisie (38) permettant la rotation de l'élément de commande (8).

13. Sonde médicale implantable selon l'une quelconque des revendications précédentes, **caractérisée en ce que** ladite structure de connexion (1) comprend en outre une partie épaissie (25) et **en ce que** ledit connecteur (7) est monté non rotatif sur ladite partie épaissie (25).
